(19) Europäisches Patentamt
European Patent Office
Office européen des brevets

(11) Numéro de publication : **0 009 487**
**B1**

(12)
# FASCICULE DE BREVET EUROPÉEN

(45) Date de publication du fascicule du brevet : **02.05.84**

(51) Int. Cl.³ : **A 61 K 31/63**, C 07 C119/06, C 07 C143/80, A 61 K 31/165

(21) Numéro de dépôt : **79900115.1**

(22) Date de dépôt : **10.01.79**

(86) Numéro de dépôt international : **PCT/CH 79/00003**

(87) Numéro de publication internationale : **WO WO/79005 (09.08.79 Gazettee 79/16)**

(54) **NOUVEAUX DERIVES DE LA BENZAMIDE.**

(30) Priorité : **17.01.78 CH 472/78**

(43) Date de publication de la demande : **16.04.80 Bulletin 80/08**

(45) Mention de la délivrance du brevet : **02.05.84 Bulletin 84/18**

(84) Etats contractants désignés : **DE FR GB LU SE**

(56) Documents cités :
**FR-M- 1 011**
**FR-M- 3 528**
**Journal of the American Pharmaceutical Association, vol. 58, no. 9, septembre 1968, page 511**
**USAN Cumulative List V.Vol. NS8, Nr. 9, Sept. 1968, p. 511**
**J.B. Stenlake: Foundations of Molecular Pharmacology, Vol. 1, (1979), Medicinal and Pharmaceutical Chemistry, p. 468**

(73) Titulaire : **DEBIOPHARM S.A.**
**Petit-Chêne 38**
**CH-1003 Lausanne (VD) (CH)**

(72) Inventeur : **PURCELL, William, Paul**
**547 Wild Cherry Cove**
**Memphis, TN 38117 (US)**
Inventeur : **PARISH, Jr., Harlie, A**
**4293 Beechcliff Lane**
**Memphis, TN 38128 (US)**

(74) Mandataire : **Meyer, Hans**
**Kirker & Cie Ingénieurs-conseil en proprieté industrielle 14, Rue du Mont-Blanc**
**CH-1211 Genève (CH)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 009 487 B1**

Jouve, 18, rue St-Denis, 75001 Paris, France

Nouveaux dérivés de la benzamide

La présente invention concerne de nouveaux dérivés de la benzamide thérapeutiquement actifs, notamment diurétiques et antihypertenseurs, de formule générale I

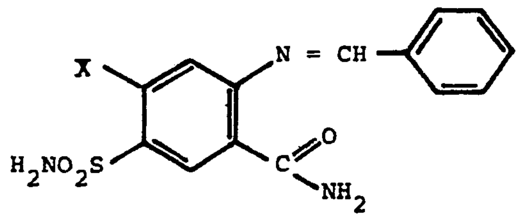

(I)

dans laquelle X représente un atome de fluor ou de chlore, et les sels de potassium de ces dérivés.

Dans la publication USAN Cumulative List, vol. NS 8, No 9 (septembre 1968), page 511, le composé N'-allyl-4-chloro-6-[(3-hydroxy-2-butylidène) amino]-m-benzènedisulfonamide est divulgé comme étant doué de propriétés diurétiques. Toutefois, une activité antihypertensive n'est pas mentionnée.

D'autre part, le brevet français N° 3528 M délivré à CIBA, Société Anonyme, décrit des dérivés de l'acide anthranilique en tant qu'agents antiinflammatoires ou hypotenseurs. On constate cependant que le seul composé cité spécifiquement dans cette publication comme hypotenseur est le morpholide de l'acide 5-(diméthyl-sulfamyl)anthranilique, dont la formule figure au haut de la page 2, colonne de droite. Or, abstraction faite de l'absence de groupement de base de Schiff et abstraction faite de la présence de substituants sur les atomes d'azote des groupements carboxamido et sulfonamido (bien que ces substituants exercent un effet pharmacologique déterminant, si l'on compare les deux formules de la page 2 du brevet CIBA), le composé cité spécifiquement comme hypotenseur n'est pas halogéné. D'ailleurs, aucun des composés cités spécifiquement par CIBA n'est halogéné. La présence possible d'un radical chloro (symbolisé par $R_5$ dans la formule générale figurant au haut de la page 1) n'est mentionnée qu'incidemment, et rien dans ce brevet ne suggère que des dérivés chlorés pourraient être pharmacologiquement actifs comme hypotenseurs.

On peut préparer les composés de l'invention en faisant réagir un composé de formule générale II :

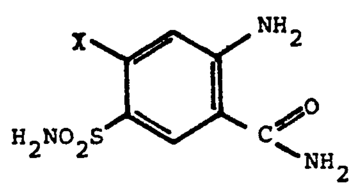

(II)

dans laquelle X représente un atome de fluor ou de chlore avec du benzaldéhyde. Cette réaction se fait de préférence en présence de chlorure de zinc, dans un solvant tel que le toluène, à une température élevée ne dépassant pas 175 °C.

Les nouveaux dérivés peuvent exister sous forme de sels alcalins, par exemple sous forme de sel de potassium, qui prend la place d'un atome d'hydrogène du groupe sulfamoyle.

## Exemple 1

a) On chauffe à reflux 2,8 g de 2-amino-4-chloro-5-sulfamoylbenzamide dans 125 ml de toluène et 45 ml de diméthylformamide comme solvant, ainsi que 1,7 g de chlorure de zinc et 5 ml de benzaldéhyde. On chauffe la solution à reflux pendant 5 h et pendant ce temps on sépare l'eau formée au cours de la réaction au moyen d'un séparateur d'eau raccordé entre le condenseur à reflux et le récipient réactionnel. Après 5 h, on chasse le solvant par distillation sous vide et on ajoute 20 ml d'éthanol au résidu huileux brun. Après repos pendant une nuit, on récolte 3,3 g de produit brut. La recristallisation dans un mélange 50/50 d'éthanol/eau donne 2,4 g (64 %) de 2-N-(benzylidènamino)-4-chloro-5-sulfamoylbenzamide qui fond à 220-230 °C et se solidifie à 260-280 °C, selon la vitesse de chauffage.

b) On ajoute 0,5 g de ce produit à 4,7 ml d'éthanol absolu contenant 0,017 g/ml d'hydroxyde de potassium. On évapore à sec la solution sous vide pour obtenir 0,4 g du sel de potassium du 2-N-(benzylidènamino)-4-chloro-5-sulfamoylbenzamide.

Le spectre IR du produit obtenu selon a) ci-dessus présente un maximum à 1 620 $cm^{-1}$ pour le groupe —N=CH—, tandis que le spectre UV présente un maximum à 279 nm. Le spectre NMR présente des adsorptions à 5,7 ppm (1 proton), 6,6 ppm (1 proton), 7,3 ppm (5 protons) et 8,2 ppm (1 proton).

## Exemple 2

2-acétamido-4-fluorotoluène (2)

A un mélange chaud (35 °C), agité de 90,0 g (0,719 mole) de 2-amino-4-fluorotoluène (1) dans 513 ml d'eau, on a ajouté en une portion 139 ml (150,4 g ; 1,473 mole) d'anhydride acétique. la température est montée à 73 °C et un solide s'est séparé (par précipitation). Le mélange fut refroidi jusqu'à température ambiante, et le solide fut récupéré dans un filtre, lavé avec de l'eau (525 ml), puis séché *in vacuo* à 80 °C ; rendement 115,7 g (96,2 %) ; m.p. 127-128 °C. La matière était encore transformable.

2-acétamido-4-fluoro-5-sulfamyltoluène (4)

A 190 ml (340,1 g ; 2,920 moles) d'acide chlorosulfonique, maintenu entre 0 et 20 °C avec un bain de glace-acétone à sec, on a ajouté par portion 95,7 g (0,572 mole) de 2-acétamido-4-fluorotoluène (2). Le bain fut retiré et 31,7 g (0,543 mole) de chlorure de sodium furent ajoutés lentement. On a noté un important moussage de chlorure d'hydrogène. La réaction ne parut pas être exothermique. Le mélange fut chauffé lentement jusqu'à atteindre 50 °C et maintenu à cette température pendant 3,75 h. Le mélange chaud fut versé dans un mélange bien agité de 600 ml d'eau et de 400 g de glace. ATTENTION : l'acide chlorosulfonique réagit vigoureusement avec l'eau. Le solide qui se sépara fut récupéré dans un filtre, lavé avec de l'eau (500 ml), puis utilisé dans la réaction suivante.

Le 2-acétamido-4-fluoro-5-chlorosulfonyltoluène (3) mouillé fut chauffé dans 650 ml d'hydroxyde d'ammoniaque concentré jusqu'à atteindre 50 °C. La solution résultante fut refroidie et extraite avec de l'acétat d'éthyle (10 × 1 000 ml). Les extraits furent combinés et concentrés *in vacuo* pour donner un solide. Celui-ci fut purifié en dissolvant le solide dans 350 ml d'une solution aqueuse à 20 % d'hydroxyde de sodium, en filtrant ce mélange, en acidifiant le filtrat avec de l'acide chlorhydrique concentré. Le précipité qui s'est séparé fut récupéré puis séché ; rendement 32,9 g (23,4 %) ; m.p. 205-207 °C ; littérature m.p. 206-208 °C.

4-fluoro-5-acide sulfamylanthranylique (6)

Un mélange de 25,0 g (0,102 mole) de sulfonamide purifié (4), 36,7 g de sulfate de magnésium, d'heptahydrate et de 560 ml d'eau fut agité et chauffé jusqu'à 80 °C. Du permanganate de potassium (48,1 g) fut ajouté par portion, maintenant la température entre 80 et 85 °C. Le mélange fut porté à 90 °C pendant 4 h, filtré à chaud, et le gâteau (aggloméré) de dioxyde de manganèse fut lavé à l'eau. Le filtrat fut acidifié avec de l'acide chlorhydrique concentré et le précipité de composé 5 fut récupéré.

Le composé mouillé 5 de la réaction ci-dessus et 4,0 g obtenus auparavant furent chauffés à reflux pendant trois heures dans 215 ml d'hydroxyde de sodium 3 N. Le mélange de réaction fut filtré et le filtrat fut acidifié avec de l'acide chlorhydrique concentré. Le solide qui se sépare fut récupéré dans un filtre, lavé à l'eau, puis séché *in vacuo* à 80 °C ; rendement 20,1 g (70,5 %).

Méthyle 2-amino-4-fluoro-5-sulfamylbenzoate (7)

Un ballon à réaction contenant 7,2 g (0,030 7 mole) de 4-fluoro-5-acide sulfamylanthranylique (6) dans 125 ml de méthanol et 10 ml d'acide sulfurique concentré fut attaché à un appareil Soxhlet contenant des tamis moléculaires 4 A immergés dans du méthanol (25 ml). Après 48 h de reflux, la solution fut versée dans de l'eau froide (500 ml) contenant 25 g de carbonate de potassium. Le solide qui se sépara fut récupéré dans un filtre et séché *in vacuo* à 80 °C ; rendement 5,6 g (73,7 %) ; m.p. 209-211 °C.

2-amino-4-fluoro-5-sulfamylbenzamide (8)

Un mélange de 4,8 g (0,019 3 mole) de méthyle 2-amino-4-fluoro-5-sulfamylbenzoate (7) dans 75 ml d'hydroxyde d'ammoniaque concentré fut porté à 50 °C pour obtenir une solution complète. La solution fut refroidie et ramenée à température ambiante, diluée avec une nouvelle solution de 75 ml d'hydroxyde d'ammoniaque concentré et on la laissa se reposer pendant 65 h. Elle fut extraite avec l'éthyle acétate (1 × 250 ml ; 4 × 500 ml) et les extraits furent combinés, séchés sur du sulfate de magnésium anhydre et concentrés *in vacuo* pour donner un solide. Celui-ci fut trituré avec 25 ml d'acétate d'éthyle pour donner 2,7 g (60,0 %) de produit pur (8).

L'acide anthranylique (6) fut récupéré en acidifiant la couche d'hydroxyde d'ammoniaque avec de l'acide chlorhydrique concentré ; récupéré 0,8 g d'acide (6).

2-N-(benzylidènamino)-4-fluoro-5-sulfamylbenzamide (9)

Un mélange de 5,4 g (0,023 2 mole) de l'amide (8), de 3,5 ml de benzaldéhyde et de 3,2 g de chlorure de zinc dans 75 ml de toluène et 30 ml de N,N-diméthylformamide fut chauffé à reflux avec enlèvement de l'eau pendant 26 h. Le solvant fut retiré *in vacuo* et le résidu marron, huileux fut dissous dans 25 ml d'une solution aqueuse à 75 % d'éthanol puis chauffé à reflux pendant 15 mn. La solution fut refroidie tout en étant agitée pendant toute la nuit et le solide qui se sépara était analytiquement pur ; rendement 3,2 g (43,2 %) ; m.p. 223-243 °C avec resolidification.

De la matière supplémentaire fut obtenue en concentrant la liqueur mère avec 20 g de colloïde de silice et en le plaçant au-dessus d'une colonne de colloïde de silice (35 cm × 4 cm). L'élution se fit avec du dichlorométhane (450 ml), dichlorométhane-éthylacétate (1 : 1) (1 050 ml), puis de l'acétate d'éthyle (600 ml). Des fractions (de 75 ml chacune) contenant du produit, selon détermination chromatographique d'une couche mince, furent combinés, concentrés in vacuo, puis dissous dans 60 ml d'éthanol et dilués avec 45 ml d'eau. Le solide qui se sépara fut récupéré et séché ; rendement 1,4 g (18,9 %).

Les composés selon l'invention sont des diurétiques et antihypertenseurs, ils ont en plus une action antagoniste de l'angiotensine et de la rénine. L'action de ces composés est prolongée dans le temps, de sorte que leur action reste efficace pendant une durée de 6 à 12 h selon les doses. Leur toxicité aiguë DL50 est inférieure à 2,4 g/kg per os et 3,0 g/kg par voie intrapéritonéale chez la souris, tandis que la dose efficace DE50 est d'environ 0,13 mg/kg. Pour le traitement chez l'homme, on utilisera des doses de 10 à 60 mg par jour en une ou plusieurs doses.

Les composés selon l'invention peuvent être administrés sous n'importe quelle forme galénique, par exemple sous forme de comprimés, capsules, gélules, suppositoires, solutions intraveineuses ou intramusculaires.

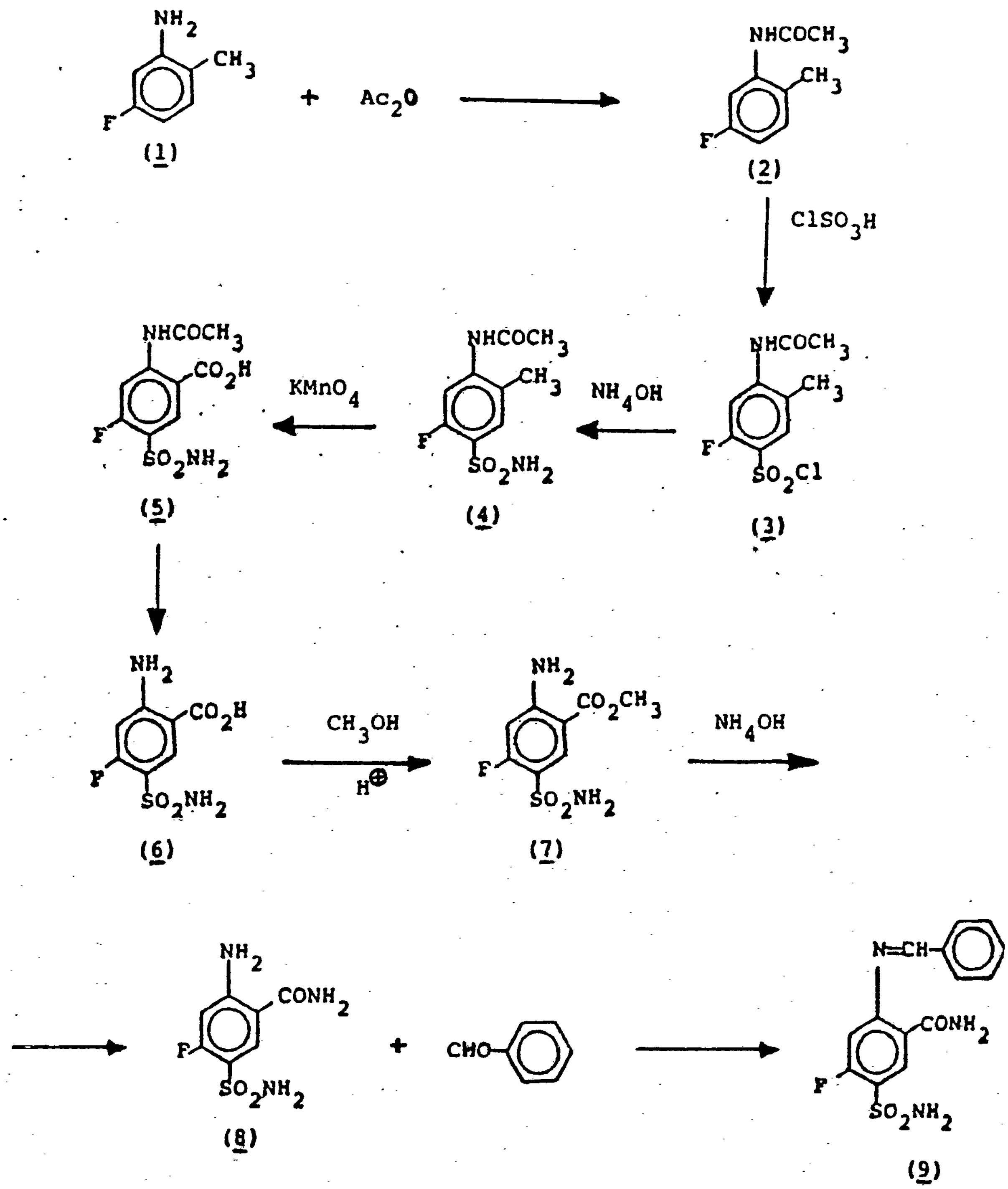

(1) (2) (3) (4) (5) (6) (7) (8) (9)

**Revendications**

1. Dérivés de la benzamide thérapeutiquement actifs, de formule générale I

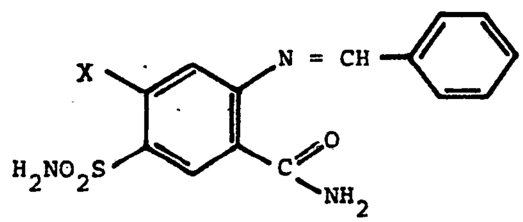

(I)

dans laquelle X représente un atome de fluor ou de chlore, et leurs sels de potassium.

2. Procédé de fabrication d'un composé selon la revendication 1, caractérisé en ce qu'on fait réagir un composé de formule générale II

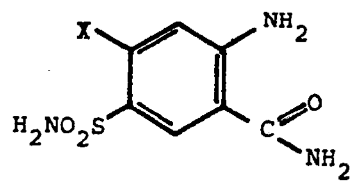

(II)

dans laquelle X représente un atome de chlore ou de fluor, avec du benzaldéhyde.

3. Procédé selon la revendication 2, caractérisé en ce qu'on effectue la réaction en présence de $ZnCl_2$ à une température ne dépassant pas 175 °C, de préférence dans du toluène.

4. Médicament, comprenant un support galénique et une quantité pharmaceutiquement active d'un composé selon la revendication 1.

**Claims**

1. Therapeutically active Benzamide derivatives of general formula I

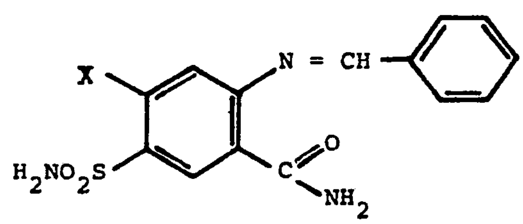

(I)

in which X represents a fluorine or chlorine atom, and their potassium salts.

2. Process for producing a compound according to claim 1, characterized by reacting a compound of general formula II

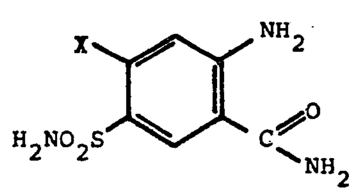

(II)

in which X represents a chlorine or fluorine atom, with benzaldehyde.

3. Process according to claim 2, characterized by carrying out the reaction in the presence of $ZnCl_2$ at a temperature not exceeding 175 °C, preferably in toluene.

4. Medicament comprising a pharmaceutical carrier and a pharmaceutically active compound of a compound according to claim 1.

**Ansprüche**

1. Therapeutisch aktive neue Benzamidderivate der allgemeinen Formel I und deren Kaliumsalze

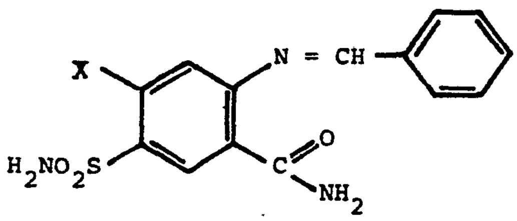

(I)

in der X ein Fluor- oder Chloratom darstellt.

2. Verfahren zur Herstellung einer Verbindung nach Anspruch 1, dadurch gekennzeichnet, dass man eine Verbindung der allgemeinen formel II

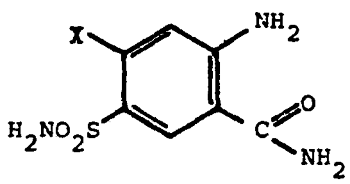

(II)

in der X ein Chlor- oder Fluoratom darstellt, mit Benzaldehyd umsetzt.

3. Verfahren nach Anspruch 2, dadurch gekennzeichnet, dass man die Reaktion in Gegenwart von $ZnCl_2$ bei einer Temperatur nich über 175 °C, vorzugsweise in Toluol, durchführt.

4. Arzneipräparat, enthaltend einen galenischen Trägerstoff und eine pharmazeutisch wirksame Menge einer Verbindung nach Anspruch 1.